# EUROPEAN PATENT APPLICATION

(11) **EP 4 631 506 A1**
(43) Date of publication of application: **15.10.2025**
(21) Application number: 23900209.0
(22) Date of filing: 14.03.2023
(51) Int. Cl.: A61K 31/497, A61K 45/00, A61P 3/10, A61P 7/12, A61P 9/00, A61P 9/12, A61P 13/12, A61P 17/00

(54) **BODY FLUID REGULATING AGENT, COMBINATION AGENT FOR BODY FLUID REGULATION AND USE OF COMPOUND**

(30) Priority: 09.12.2022 JP 2022197009
(71) Applicant: Asahi Pharma Co., Ltd., Tsukuba City, Ibaraki, 3050031 (JP)
(72) Inventor: NII Keni, Tsukuba-shi, Ibaraki 305-0031 (JP); WANG Shiyue, Dongyang City, Zhejiang Province 322103 (CN)
(74) Representative: V.O.
(86) International application number: PCT/JP2023/009774
(87) International publication number: WO 2024/122076

(57) **Abstract**

A body fluid regulator contains a compound represented by Formula (I), an ester of the compound, or a salt of the compound,

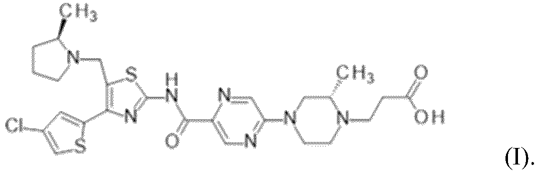

## Description

### Technical Field

The present invention relates to a body fluid regulator, a body fluid regulating combination agent, and a use of compounds.

### Background Art

Excessive retention of body fluids is a symptom associated with many diseases such as heart failure, hypertension, cirrhosis, renal disease, lymphedema, and lipedema. In particular, patients who have lost residual renal function urinate 200 mL or less per day, resulting in diminished efficacy of diuretics. Treatments for the loss of residual renal function are dialysis and kidney transplantation. However, in approximately 1/3 of the patients, fluid removal by dialysis is insufficient (see Non Patent Literature 1). Additionally, there are limited donors for kidney transplantation, and applicable cases for kidney transplantation are rare. Hence, there is a clinical demand for body fluid regulators other than diuretics, dialysis therapy, and kidney transplantation.

Localized interstitial fluid retention (edema) is a common clinical sign of both lymphedema and lipedema. Lymphedema is caused by lymphatic stasis, leading to the accumulation of body fluid around blood vessels and in subcutaneous tissues. For excess interstitial fluid in lipedematous tissues, several potential causes have been reported, including increased vascular permeability due to microvascular damage and insufficient drainage of interstitial fluid via lymphatic vessels (see Non Patent Literature 2). Sweat glands receive blood supply from cutaneous vascular perforators, and blind-ended lymphatic capillaries running in parallel with the blood supply originate from a dermal papilla and discharge interstitial fluid into a venous system. Sweating improves excessive interstitial fluid in subcutaneous tissues associated with lymphedema and lipedema caused by increased vascular permeability and insufficient lymphatic drainage.

According to Non Patent Literature 3, sweating as a physiological phenomenon has a fluid excretion capacity that exceeds that of urination. Thus, sweating is expected to serve as a new body fluid regulation method alternative to diuretics, dialysis therapy, and kidney transplantation. Detailed studies have revealed that sweating is regulated through M₃ muscarinic receptors. As disclosed in Non Patent Literature 4, sweating tests using pilocarpine, a non-selective M₃ muscarinic receptor agonist, are widely used clinically as diagnostic methods.

### Citation List

### Patent Literature

Patent Literature 1: WO 2015/186821 A

### Non Patent Literature

Non Patent Literature 1: Michelle M.Y. Wong, et al., Interdialytic Weight Gain: Trends, Predictors, and Associated Outcomes in the International Dialysis Outcomes and Practice Patterns Study (DOPPS), Am J Kidney Dis., 69 (3), 367-379, 2017
Non Patent Literature 2: Allen M, Michael Schwartz M, and Herbst K. L., Interstitial Fluid in Lipedema and Control Skin, Women's Health Reports, 1 (1), 480-487, 2020
Non Patent Literature 3: Lindsay B. Baker, Physiology of sweat gland function: The roles of sweating and sweat composition in human health, Temperature, 6 (3), 211-259, 2019
Non Patent Literature 4: Pamela B. Davis, Cystic Fibrosis Since 1938, American Journal of Respiratory and Critical Care Medicine, 173, 475-482, 2006

### Summary of the Invention

### Problems to be Solved by the Invention

There are no known agonists that selectively stimulate M₃ muscarinic receptors, and side effects due to non-selective muscarinic receptor stimulation are a concern. Moreover, stimulation by agonists may lead to constant sweating, impacting daily life. More flexible temporal control over sweating would allow for the improvement of excessive fluid retention without compromising quality of life.

In view of the above, the present invention aims to provide a body fluid regulator, a body fluid regulating combination agent, and a use of compounds that enable more flexible temporal control of sweating.

### Solutions to Problems

The compound disclosed in Patent Literature 1 is a positive allosteric modulator (PAM) of the M₃ muscarinic receptors. The present inventors have found that although this compound alone does not affect sweating, it promotes sweating in a dosage-dependent manner when administered in combination with an M₃ muscarinic receptor agonist, thereby completing the present invention.

A body fluid regulator according to a first aspect of the present invention contains: a compound represented by Formula (I), an ester of the compound, or a salt of the compound,

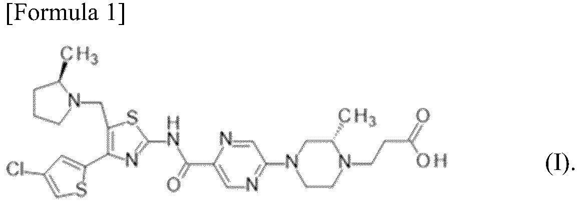

The body fluid regulator according to the first aspect of the present invention may be administered in combination with a diuretic to a subject.

The body fluid regulator according to the first aspect of the present invention may be administered in combination with an M₃ muscarinic receptor agonist to a subject.

The body fluid regulator according to the first aspect of the present invention may be administered in combination with a thermal stimulation or a thermogenesis stimulation applied to a subject.

The subject may suffer from a disease associated with excessively retained body fluid.

The disease may be heart failure, hypertension, cirrhosis, renal disease, nephrotic syndrome, renal failure, diabetes, drug-induced excess body fluid retention, lipedema, or lymphedema.

When the disease is renal failure, the body fluid regulator according to the first aspect of the present invention may be used to delay an initiation of dialysis therapy.

The subject may suffer from a disease accompanied by skin dryness or pruritus.

The disease may be senile xerosis, atopic dermatitis, psoriasis, ichthyosis, radiation dermatitis, actinic keratosis, senile keratosis, renal disease, renal failure, or diabetes.

A body fluid regulating combination agent according to a second aspect of the present invention contains: a compound represented by Formula (I), an ester of the compound, or a salt of the compound; and a diuretic,

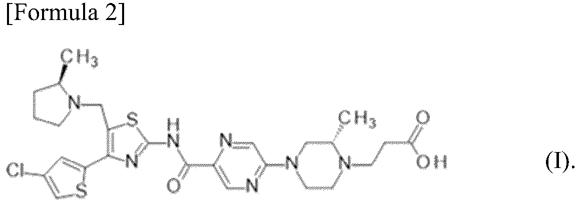

A body fluid regulating combination agent according to a third aspect of the present invention contains: a compound represented by Formula (I), an ester of the compound, or a salt of the compound; and an M₃ muscarinic receptor agonist,

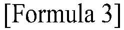

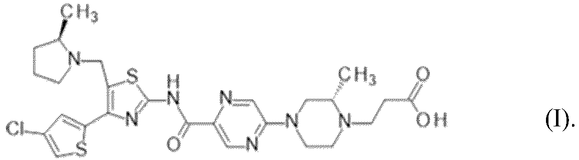

A use of compounds according to a fourth aspect of the present invention is a use of a compound represented by Formula (I), an ester thereof, or a salt thereof for production of a body fluid regulator,

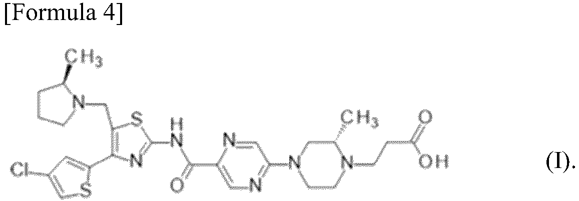

### Effects of the Invention

According to the present invention, it is possible to perform more flexible temporal control of sweating.

### Brief Description of Drawings

Fig. 1 is a diagram showing a sweating volume quantified in Test Example 1.
Fig. 2 is a diagram showing a sweating volume quantified in Example 1.
Fig. 3 is a diagram showing a sweating volume quantified in Example 2. Here, Fig. 3A, Fig. 3B, Fig. 3C, and Fig. 3D respectively represent the results of administration of 0 mg/kg, 10 mg/kg, 30 mg/kg, and 100 mg/kg of a compound.

### Description of Embodiments

Embodiments of the present invention will be described with reference to the drawings. However, the present invention is not limited to the embodiments and drawings described below. Furthermore, in the following embodiments, expressions such as "having", "including", or "containing" also encompass the meanings of "consisting of' or "made of'.

### (Embodiments)

The body fluid regulator according to the present embodiment includes a compound represented by Formula (I), an ester thereof, or a salt thereof.

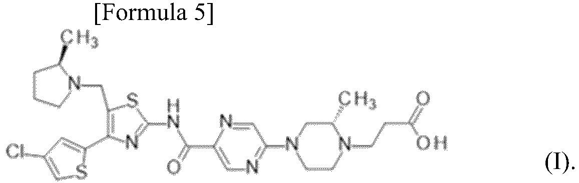

The compound is a PAM for the M₃ muscarinic receptors. Generally, PAMs are compounds that bind to an allosteric site different from a ligand-binding site and mainly induce conformational changes in a receptor, thereby increasing the binding affinity between an endogenous agonist and the receptor and modulating a signal level of the agonist. PAMs themselves do not exhibit agonist activity in vivo, but enhance the activity of agonists.

In the present description, the compound of Formula (I) may be represented in only one isomeric form; however, the compound according to the present embodiment includes other isomers, including separated isomers and mixtures thereof. Furthermore, the compound of Formula (I) may include optical isomers due to asymmetric carbon atoms or axial chirality. The compound according to the present embodiment also includes isolated optical isomers of the compound of Formula (I), as well as mixtures thereof.

Additionally, the present embodiment includes pharmaceutically acceptable prodrugs of the compound represented by Formula (I). A pharmaceutically acceptable prodrug is a compound that has a group convertible to an amino group, a hydroxyl group, a carboxyl group or the like through solvolysis or under physiological conditions.

The salts of the compound represented by Formula (I) are not particularly limited as long as they are pharmacologically acceptable, and may be either acidic or basic salts. Examples of such salts include: alkali metal salts such as lithium, sodium, and potassium salts; alkaline earth metal salts such as magnesium and calcium salts; inorganic acid salts such as hydrochloride, hydrobromide, hydroiodide, sulfate, nitrate, and phosphate salts; and organic acid salts such as formate, acetate, oxalate, propionate, hexanoate, cyclopentylpropionate, glycolate, pyruvate, lactate, malonate, succinate, malate, fumarate, tartrate, dibenzoyltartrate, ditoluoyltartrate, citrate, benzoate, o-(4-hydroxybenzoyl)benzoate, cinnamate, mandelate, methanesulfonate, ethanesulfonate, 1,2-ethanedisulfonate, 2-hydroxyethanesulfonate, benzenesulfonate, p-chlorobenzenesulfonate, 2-naphthalenesulfonate, p-toluenesulfonate, aspartate, camphorsulfonate, gluceptate, 3-phenylpropionate, trimethylacetate, tert-butylacetate, lauryl sulfate, gluconate, glutamate, hydroxynaphthoate, salicylate, stearate, trifluoroacetate (TFA), maleate, dimaleate, and muconate. Preferably, the body fluid regulator according to the present embodiment contains the dimaleate salt of the compound represented by Formula (I).

The esters of the compound represented by Formula (I) are not particularly limited as long as they are pharmacologically acceptable. Examples of the esters include carbonate esters, phosphate esters, nitrate esters, sulfate esters, borate esters, and sulfonate esters.

Furthermore, the present embodiment includes various hydrates, solvates, and crystal polymorphs of the compound represented by Formula (I) and salts thereof. The present invention also includes compounds labeled with various radioactive or nonradioactive isotopes.

The compound of Formula (I), as well as the esters and salts thereof, can be synthesized using various known synthetic methods based on their basic structure or the type of substituent groups. **In** such cases, depending on the type of functional group, it may be effective during synthesis to replace the functional group with a suitable protecting group (a group readily convertible to the functional group) at the stage from the starting material to the intermediate. The desired compound can be obtained by introducing the protecting group, conducting the reaction, and then removing the protecting group as necessary.

Here, a representative method for synthesizing the compound of Formula (I) and its starting material which is a compound of Formula (a) will be described. The method for synthesizing the compound is not limited to the examples described below.

### (First Synthetic Method)

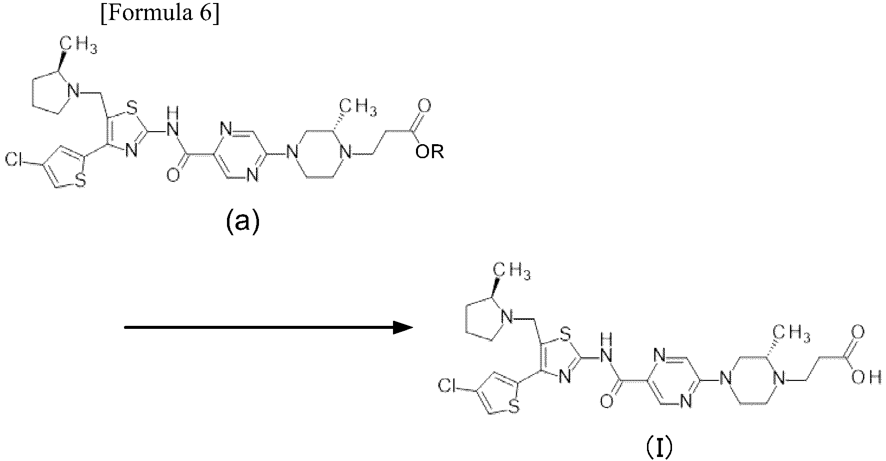

(Here, R represents a C1-C6 alkyl group. The same applies hereinafter.)

The first synthetic method involves deprotecting the compound of Formula (a) to synthesize the compound of Formula (I). In the first synthetic method, the compound of Formula (a) and a deprotecting reagent are used in equimolar amounts or with one in excess, and stirred in a solvent which is inert to the reaction or under solvent-free conditions, under cooling to reflux heating conditions, typically for 0.1 hours to 5 days. Examples of the solvents used here include, but are not limited to, alcohols such as methanol, ethanol, and n-propanol, as well as N,N-dimethylformamide and tetrahydrofuran. Examples of deprotecting reagents include, but are not limited to, bases such as sodium hydroxide solution and potassium hydroxide solution, and acids such as hydrochloric acid and trifluoroacetic acid.

### (Second Synthetic Method)

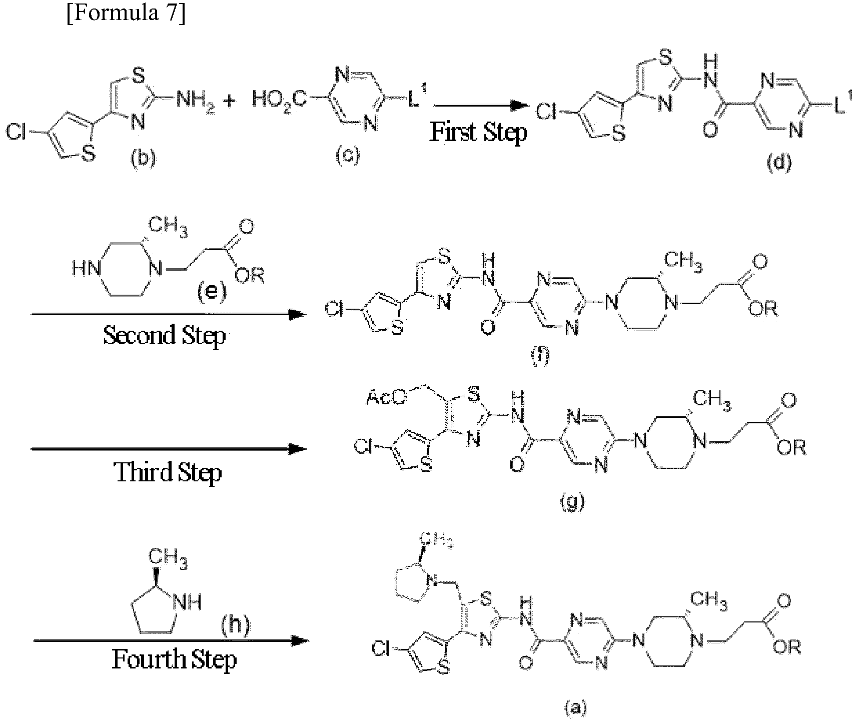

(Here, L¹ represents a leaving group. The same applies hereinafter.)

The second synthetic method is a method for synthesizing the compound of Formula (a), which is the starting material of the compound of Formula (I). Here, examples of L¹ include Cl.

### (First Step)

In this step, a compound of Formula (d) is obtained from a compound of Formula (b) and a compound of Formula (c) through an amidation reaction. In this reaction, the compound of Formula (b) and the compound of Formula (c) are used in equal amounts or with one in excess, and the mixture thereof is stirred in the presence of a condensing agent in a solvent which is inert to the reaction under cooling to heating conditions, preferably in a temperature range from -20°C to 60°C for a period typically ranging from 0.1 hours to 5 days. Examples of the solvents used here include, but are not limited to, aromatic hydrocarbons such as benzene, toluene, and xylene, halogenated hydrocarbons such as dichloromethane, 1,2-dichloroethane, and chloroform, ethers such as diethyl ether, tetrahydrofuran, dioxane, 1,2-dimethoxyethane, and cyclopentyl methyl ether; N,N-dimethylformamide, dimethyl sulfoxide, ethyl acetate, acetonitrile, water, and mixtures thereof. Examples of condensing agents include: 1-(3-dimethylaminopropyl)-3-ethylcarbodiimide, hydrochloride salts of 1-(3-dimethylaminopropyl)-3-ethylcarbodiimide, dicyclohexylcarbodiimide, 1,1'-carbonyldiimidazole, diphenylphosphoryl azide, phosphorus oxychloride, and N-[({[(1Z)-1-cyano-2-ethoxy-2-oxoethylidene]amino}oxy)(morpholin-4-yl)methylene]-N-methylmethanaminium hexafluorophosphate (COMU). It may be preferable to use an additive (for example, 1-hydroxybenzotriazole) in the reaction. The reaction may also be conducted in the presence of organic bases such as triethylamine, N,N-diisopropylethylamine, and N-methylmorpholine, or inorganic bases such as potassium carbonate, sodium carbonate, and potassium hydroxide.

Alternatively, a method may be employed in which a carboxylic acid (c) is converted into a reactive derivative and then reacted with an amine (b). Examples of reactive derivatives of carboxylic acids include acid halides obtained by reacting carboxylic acids with halogenating agents such as phosphorus oxychloride and thionyl chloride, mixed acid anhydrides obtained by reacting carboxylic acids with isobutyl chloroformate, etc., and active esters obtained through condensation of carboxylic acids with 1-hydroxybenzotriazole, etc. The reaction of these reactive derivatives with the compound (b) can be conducted in a solvent inert to the reaction, such as halogenated hydrocarbons, aromatic hydrocarbons, and ethers, under cooling to heating conditions, preferably in a temperature range from -20°C to 60°C.

### (Second Step)

In this step, a compound of Formula (f) is synthesized by reacting a compound of Formula (d) with a compound of Formula (e). In this reaction, the compound (d) and the compound (e) are used in equal amounts or with one in excess, and the mixture thereof is stirred in a solvent which is inert to the reaction or under solvent-free conditions, under cooling to reflux heating conditions, preferably in a temperature range from 0°C to 80°C for a period typically ranging from 0.1 hours to 5 days. Examples of the solvents used here include, but are not limited to, aromatic hydrocarbons such as benzene, toluene, and xylene, ethers such as diethyl ether, tetrahydrofuran, dioxane, and 1,2-dimethoxyethane, halogenated hydrocarbons such as dichloromethane, 1,2-dichloroethane, and chloroform, N,N-dimethylformamide, N-methylpyrrolidone, dimethyl sulfoxide, ethyl acetate, acetonitrile, and mixtures thereof. The reaction may also be conducted in the presence of organic bases such as triethylamine, N,N-diisopropylethylamine, and N-methylmorpholine, or inorganic bases such as potassium carbonate, sodium carbonate, and potassium hydroxide.

### (Third Step)

In this step, an acetyloxymethyl group is introduced at the 5-position of the thiazole ring of the compound of Formula (f) to synthesize the compound of Formula (g). For example, in this reaction, the compound of Formula (f) is reacted with an aqueous solution of formaldehyde or paraformaldehyde in acetic acid solvent, at room temperature or under heating conditions, or at room temperature or under reflux conditions. Alternatively, instead of using acetic acid solvent, the reaction may be conducted in a solvent which is inert to the reaction, such as halogenated hydrocarbons, aromatic hydrocarbons, or ethers, with acetic acid added. Furthermore, acetic anhydride may be added to the reaction.

### (Fourth Step)

In this step, the compound of Formula (a) is synthesized by reacting a compound of Formula (g) with a compound of Formula (h) under basic conditions. For example, in this reaction, the compound of Formula (g) is reacted with the compound of Formula (h) in an organic solvent which is inert to the reaction, such as halogenated hydrocarbons, aromatic hydrocarbons, ethers, esters, acetonitrile, N,N-dimethylformamide, dimethyl sulfoxide, or N-methylpyrrolidone, in the presence of an organic base such as triethylamine or N,N-diisopropylethylamine. Alternatively, the compound of Formula (h) may be used in excess instead of using an organic base. The reaction may be conducted under cooling conditions or at room temperature, at room temperature or under heating conditions, or at room temperature or under reflux conditions. Furthermore, by adding the compound of Formula (h) to the reaction mixture from the third step, the compound of Formula (a) can be obtained directly without isolating the compound of Formula (g).

The compound of Formula (I) may be isolated and purified as a free compound, a salt thereof, a hydrate thereof, a solvate thereof, or a crystal polymorph thereof. The salts of the compound of Formula (I) may also be synthesized by conventional methods. Isolation and purification may be performed by standard chemical operations such as extraction, fractional crystallization, and various types of chromatographic separation. Various isomers may be synthesized by selecting appropriate compounds as starting materials or may be separated by utilizing differences in the physicochemical properties of the isomers. For example, optical isomers may be obtained by general optical resolution methods for racemates (for example, fractional crystallization via the formation of diastereomeric salts with optically active bases or acids, and chromatography using chiral columns or the like). Furthermore, optical isomers may also be synthesized from optically active compounds as starting materials.

The body fluid regulator according to the present embodiment contains, as an active ingredient, the compound represented by Formula (I), the esters thereof, or the salts thereof (hereinafter also referred to as "compound, ester, or salt"), and may further contain other pharmacologically acceptable ingredients. Other pharmacologically acceptable ingredients include, for example, excipients, lubricants, binders, and disintegrants in solid formulations, or solvents, solubilizers, suspending agents, isotonic agents, buffering agents, and analgesics in liquid formulations. Additionally, if necessary, additives such as preservatives, antioxidants, colorants, and sweeteners may be blended into the body fluid regulator.

Examples of excipients include lactose, sucrose, D-mannitol, starch, crystalline cellulose, and light anhydrous silicic acid. Examples of lubricants include magnesium stearate, calcium stearate, talc, and colloidal silica. Examples of binders include crystalline cellulose, sucrose, D-mannitol, dextrin, hydroxypropyl cellulose, hydroxypropyl methylcellulose, and polyvinylpyrrolidone. Examples of disintegrants include starch, carboxymethyl cellulose, calcium carboxymethyl cellulose, croscarmellose sodium, and sodium carboxymethyl starch.

Examples of solvents include water for injection, alcohol, propylene glycol, and macrogol. Examples of solubilizers include polyethylene glycol, propylene glycol, D-mannitol, benzyl benzoate, ethanol, tris-aminomethane, cholesterol, triethanolamine, sodium carbonate, and sodium citrate. Examples of suspending agents include surfactants and hydrophilic polymers such as stearyl triethanolamine, sodium lauryl sulfate, laurylaminopropionic acid, lecithin, benzalkonium chloride, benzethonium chloride, glyceryl monostearate, polyvinyl alcohol, polyvinylpyrrolidone, sodium carboxymethyl cellulose, methyl cellulose, hydroxymethyl cellulose, hydroxyethyl cellulose, and hydroxypropyl cellulose.

Examples of isotonic agents include sodium chloride, glycerin, and D-mannitol. Examples of buffering agents include buffer solutions of phosphates, acetates, carbonates, and citrates. Examples of analgesics include benzyl alcohol. Examples of preservatives include esters of para-hydroxybenzoic acid, chlorobutanol, benzyl alcohol, phenethyl alcohol, dehydroacetic acid, and sorbic acid. Examples of antioxidants include sulfites and ascorbic acid.

The body fluid regulator according to the present embodiment is administered to a human or a non-human animal. Preferred animals include mammals, more specifically dogs, cats, cattle, pigs, horses, sheep, and deer. The administration route of the body fluid regulator to humans or animals is not particularly limited. The body fluid regulator may be administered as an injection, but is preferably used as an oral dosage form. The body fluid regulator may be synthesized by known methods and provided in forms such as solutions, tablets, granules, fine granules, powders, tablets, and capsules.

The dosage of the body fluid regulator is appropriately determined based on factors such as the age, weight, and condition of the human or animal who is the subject of administration. The body fluid regulator is administered so that the compound, ester, or salt is delivered in an effective amount. An effective amount refers to the amount necessary to delay, inhibit, prevent, reverse, or cure the progression of symptoms associated with diseases involving excessively retained body fluid in the subject of administration.

The body fluid regulator according to the present embodiment can enhance the effects of diet-induced thermogenesis and promote sweating via stimulation of M₃ muscarinic receptors. Accordingly, even in the absence of exogenous thermal stimulation or thermogenesis stimulation, the body fluid regulator alone, when administered to a subject, can induce a certain level of sweating. Since the body fluid regulator has the characteristic of stimulation-dependent promotion of sweating, the duration of sweating can be controlled by adjusting the timing of diet-induced thermogenesis or the like.

Preferably, the body fluid regulator according to the present embodiment is administered in combination with a diuretic or an M₃ muscarinic receptor agonist to a subject. For example, an additive effect may be achieved by co-administering the body fluid regulator with a diuretic whose pharmacological effect is diminished due to impaired renal function. Furthermore, by co-administering the body fluid regulator with a threshold dosage of an M₃ muscarinic receptor agonist, sweating can be induced while avoiding the adverse effects and inappropriate timing associated with the M₃ muscarinic receptor agonist. In addition, by co-administering the body fluid regulator with a diuretic or an M₃ muscarinic receptor agonist, it is possible to induce sweating in patients with decreased physical strength, such as those with uremia, without requiring physical exercise, bathing, or the like.

The diuretic is not particularly limited, and examples of the diuretic include: loop diuretics such as furosemide, bumetanide, torasemide, piretanide, and azosemide; thiazide diuretics such as trichlormethiazide, hydrochlorothiazide, and bendroflumethiazide; thiazide-like diuretics such as chlortalidone, mefruside, indapamide, tripamide, and methyclothiazide; potassium-sparing diuretics such as spironolactone, triamterene, and potassium canrenoate; carbonic anhydrase inhibitor diuretics such as acetazolamide; osmotic diuretics such as isosorbide, concentrated glycerin, fructose, and D-mannitol; and agents such as mozavaptan hydrochloride.

The M₃ muscarinic receptor agonist may be an M₃ muscarinic receptor agonist that directly activates the M₃ muscarinic receptors, a cholinesterase inhibitor that indirectly stimulates the M₃ muscarinic receptors, or the like. Examples of the M₃ muscarinic receptor agonist include: pilocarpine, cevimeline, carbachol, bethanechol, acetylcholine, methacholine, and carpronium. Examples of the cholinesterase inhibitor include: donepezil, galantamine, rivastigmine, and distigmine.

The term "co-administering" means administering the body fluid regulator according to the present embodiment and the diuretic or the M₃ muscarinic receptor agonist to the same patient within a defined time period. In co-administration, the body fluid regulator and the diuretic or the M₃ muscarinic receptor agonist may be administered simultaneously, or administered separately at different times, e.g., one of them is administered while the effect of the other is still remaining. In co-administration, the administration routes for the body fluid regulator and the diuretic or the M₃ muscarinic receptor agonist may be the same or different. Examples of modes of co-administration include: administration of a single formulation prepared by preformulating the body fluid regulator and a diuretic or an M₃ muscarinic receptor agonist prior to administration; simultaneous administration of two formulations which are formulated separately as the body fluid regulator and the diuretic or the M₃ muscarinic receptor agonist via the same administration route; time-staggered administration of the two formulations via the same administration route; simultaneous administration of the two formulations via different administration routes; and time-staggered administration of the two formulations via different administration routes. For example, in co-administration, the body fluid regulator and the diuretic or the M₃ muscarinic receptor agonist may be administered over a predetermined period according to a single regimen that defines the dosage and administration method for each.

The body fluid regulator according to the present embodiment may also be administered in combination with a thermal stimulation or a thermogenesis stimulation applied to the subject. The term "thermal stimulation" refers to physical stimulation that applies heat, such as: having the subject use a heating appliance, a warming device, a foot warmer or the like; having the subject take a warm bath or be exposed to warm water; having the subject use a sauna; irradiating the subject with infrared rays; and setting the environmental temperature of the subject to a high level. The term "thermogenesis stimulation" refers to stimulation that induces metabolic heat production, such as food intake or physical exercise by the subject. The timing of thermal stimulation or thermogenesis stimulation to the subject is not particularly limited, as long as the thermal stimulation or thermogenesis stimulation occurs during the effective period of the body fluid regulator which is administered to the subject, and may be before or after administration of the body fluid regulator to the subject. With the body fluid regulator, the amount and duration of sweating can be controlled by adjusting the intensity of the thermal stimulation or thermogenesis stimulation and the duration of the thermal stimulation or thermogenesis stimulation.

The subject may have a disease associated with excessively retained body fluid. Examples of excessively retained body fluid include ascites and edema (for example, cardiac edema, renal edema, hepatic edema, drug-induced edema, lipedema, lymphedema, and edema caused by peripheral vascular disorders). Examples of such diseases include: heart failure, hypertension, cirrhosis, renal disease, nephrotic syndrome, renal failure, diabetes, drug-induced excess body fluid retention, lipedema, and lymphedema.

In the determination criteria for initiating dialysis therapy for renal failure, body fluid retention (systemic edema, severe hypoproteinemia, or pulmonary edema) and body fluid abnormality (unmanageable electrolyte and acid-base balance abnormalities) are critical considerations. By promoting sweating, the excretion of water and electrolytes contained in sweat is expected to improve body fluid retention and body fluid abnormality, thereby delaying the initiation of dialysis therapy. Therefore, the body fluid regulator according to the present embodiment may be used to delay the initiation of dialysis therapy in a subject with renal failure as a disease associated with excessively retained body fluid. It should be noted that "delay the initiation of dialysis therapy" refers to postponing the commencement of dialysis therapy in the subject compared to the timing in a case without administration of the body fluid regulator.

Xerosis, which is observed in aging and in many diseases, is primarily caused by a decrease in the water content of the epidermis. Since there are no blood vessels in the epidermis, moisture in the epidermis is maintained by moisture transfer from the dermis, natural moisturizing factors within the epidermis, and the physical barrier of the outer stratum corneum (D. Barco and A. Gimenez-Arnau, Xerosis: a Dysfunction of the Epidermal Barrier, Actas Dermosifiliogr, 99, 671-82, 2008). Sweat first increases the water content of the epidermis before it reaches the skin surface (Gerrett N., et al., Sweat from gland to skin surface: production, transport, and skin absorption, J Appl Physiol 125, 459-469, 2018). Natural moisturizing factors such as urea, amino acids, lactic acid, and inorganic salts contained in sweat are also replenished. For example, urea, which is one of the natural moisturizing factors contained in sweat, is a hygroscopic molecule that plays an important role in hydrating the stratum corneum. Furthermore, urea regulates gene expression related to the differentiation and proliferation of keratinocyte and the production of antimicrobial peptide, thereby improving skin barrier functions including antimicrobial defense. Thus, urea is essential for maintaining the integrity of the epithelial layer (Piquero-Casals J., et al., Urea in Dermatology: A Review of its Emollient, Moisturizing, Keratolytic, Skin Barrier Enhancing and Antimicrobial Properties, Dermatol Ther. (Heidelb), 11, 1905-1915, 2021). In other words, the promotion of sweating induced by the body fluid regulator according to the present embodiment improves skin dryness by replenishing both water and moisturizing ingredients. Accordingly, the subject may be suffering from a disease accompanied by skin dryness or pruritus. Examples of such diseases include: senile xerosis, atopic dermatitis, psoriasis, ichthyosis, radiation dermatitis, actinic keratosis, senile keratosis, (chronic) renal disease, renal failure, and diabetes.

Many drugs are excreted via the kidneys. The administration of drugs to a subject with impaired renal function presents problems in drug metabolism and, consequently, safety, such as elevated blood concentrations. As shown in Example 3 described later, the compound, ester, or salt has the characteristic of a low renal excretion rate. Therefore, the body fluid regulator according to the present embodiment is safe and facilitates blood concentration control when used for: excretion of excessively retained body fluid in subjects with reduced renal function, such as those with renal disease, renal failure, diabetic nephropathy, or resistant renal disease; and improvement of dryness, repair of skin barrier, and suppression of itching in skin dryness and pruritus associated with renal failure or chronic renal disease.

In addition, thirst is the major reason that subjects with reduced renal function cannot comply with fluid intake restrictions. Excessive fluid intake beyond the restrictions by such subjects contributes to the development of body fluid retention. The compound promotes salivary secretion, as disclosed in WO 2022/054965 A. Improvement of thirst through promotion of salivary secretion contributes to better compliance with fluid intake restrictions in subjects with reduced renal function.

In another embodiment, a body fluid regulating combination agent is provided, containing: the compound, ester, or salt described above, and a diuretic or an M₃ muscarinic receptor agonist. The body fluid regulating combination agent is a combination of the compound, ester, or salt as an active ingredient with a diuretic or an M₃ muscarinic receptor agonist. The body fluid regulating combination agent may be administered as a combined formulation combining the compound, ester, or salt with a diuretic or an M₃ muscarinic receptor agonist.

The term "body fluid regulating combination agent" encompasses: a combined formulation in which the compound, ester, or salt and the diuretic or the M₃ muscarinic receptor agonist are formulated together, and a kit in which each of the agents is prepared separately as a formulation. In the case where the body fluid regulating combination agent is a combined formulation, the compound, ester, or salt and the diuretic, or the compound, ester, or salt and the M₃ muscarinic receptor agonist can be formulated to prepare the combined formulation using conventional methods for preparing combined formulations by mixing a plurality of drugs. The combined formulation may also contain optional ingredients other than the compound, ester, or salt and the diuretic or the M₃ muscarinic receptor agonist. These optional ingredients are other pharmacologically acceptable ingredients which may be contained in the body fluid regulator described above. The combined formulation may be in any form, such as liquid, solid, semi-solid, or powder. There is no particular limitation on the blending ratio between the compound, ester, or salt and the diuretic or the M₃ muscarinic receptor agonist, provided that each can exert its respective effect.

In a case where the body fluid regulating combination agent according to the present embodiment is a kit including: a first formulation containing the compound, ester, or salt, and a second formulation containing the diuretic or the M₃ muscarinic receptor agonist, the forms of the first formulation and the second formulation may be the same or different. Furthermore, the first formulation and the second formulation may be formulations having the same administration route or administration method or formulations having different administration routes or administration methods. For example, the first formulation and the second formulation may both be oral or parenteral formulations, the first formulation may be an oral formulation and the second formulation a parenteral formulation, or the first formulation may be a parenteral formulation and the second formulation an oral formulation. The first formulation may further contain optional ingredients in addition to the compound, ester, or salt. The second formulation may further contain optional ingredients in addition to the diuretic or the M₃ muscarinic receptor agonist. These optional ingredients are other pharmacologically acceptable ingredients which may be contained in the body fluid regulator described above. Furthermore, the second formulation may contain both the diuretic and the M₃ muscarinic receptor agonist.

In another embodiment, there is provided the use of the compound represented by Formula (I), the ester thereof, or the salt thereof for the production of the body fluid regulator. In another embodiment, a method of regulating body fluid is provided. The method includes a step of administering the compound, the ester thereof, or the salt thereof to a subject suffering from a disease associated with excessive retention of body fluid. In another embodiment, there is provided the compound, the ester thereof, or the salt thereof for use in the treatment of a disease associated with excessive retention of body fluid.

The following examples will further illustrate the present invention in detail, but the present invention is not limited by the examples.

### Examples

### [Test Example 1: Threshold Determination of Pilocarpine-Induced Sweating Effect]

CD(SD) rats (male, 7 weeks old, supplied by THE JACKSON LABORATORY JAPAN, INC.) were acclimated for 1 week. During the rearing period, the rats were fed CRF-1 (supplied by Oriental Yeast Co., Ltd.) sterilized by gamma irradiation at 30 kGy or more, as well as drinking water which was tap water with chlorine added (4 ppm to 6 ppm). Food and drinking water were consumed ad libitum. Pilocarpine hydrochloride (supplied by FUJIFILM Wako Pure Chemical Corporation) was dissolved in physiological saline immediately before use to prepare a dosing solution. The dosage was 0 (physiological saline), 1, 3 or 10 mg/kg (n = 3 to 4).

The rats were intraperitoneally injected with 0.5 ml of a triple anesthetic mixture per 100 g of rat body weight. After the disappearance of righting reflex, physiological saline or the above-mentioned dosing solution was administered subcutaneously, and 5 minutes later, a filter paper whose weight had been measured in advance was placed on the palm of each limb and tightly wrapped with plastic wrap or the like. Sweat secreted over a 30-minute period was absorbed and collected on the filter paper.

### (Results)

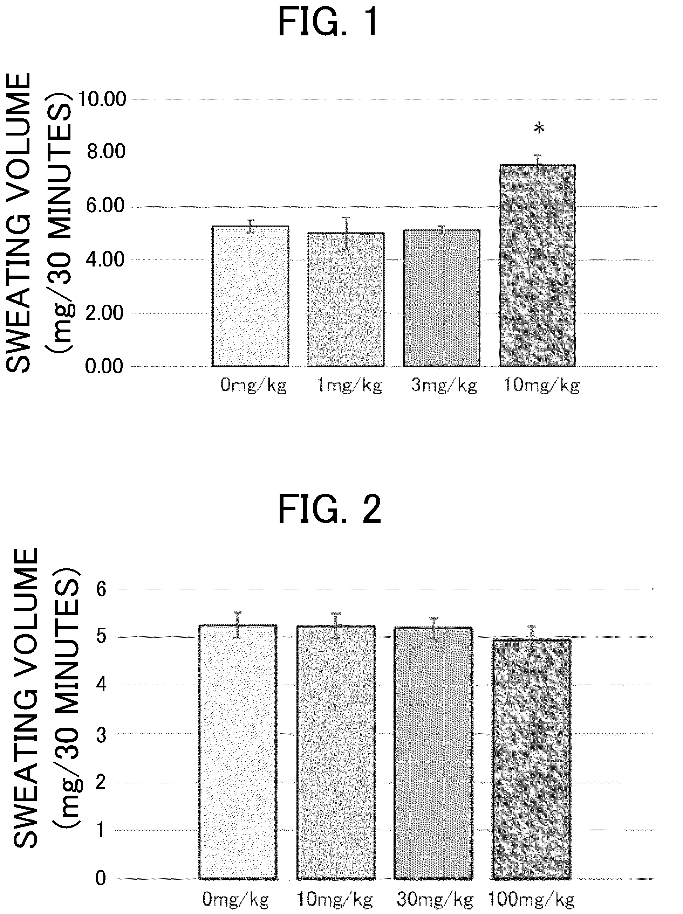

The weight of sweat collected over the 30-minute period (sweating volume) is shown in Fig. 1. A significantly higher sweating effect was observed in the 10 mg/kg group compared to the 0 mg/kg group (one-tailed T-test, *p<0.05). 3 mg/kg was determined to be the threshold for pilocarpine sweating effect.

### [Example 1: Evaluation of Sweating Effect of Compound]

The compound represented by Formula (I) (3-[(2S)-4-(5-{[4-(4-chlorothiophen-2-yl)-5-{[(2R)-2-methylpyrrolidin-1-yl]methyl}-1,3-thiazol-2-yl]carbamoyl}pyrazin-2-yl)-2-methylpiperazin-1-yl]propanoic acid dimaleate) was evaluated for its sweating effect.

The compound was suspended in 0.5 w/v % methylcellulose 400 solution (hereinafter referred to as MC solution, supplied by FUJIFILM Wako Pure Chemical Corporation) immediately before use to prepare a dosing solution. The dosage was 0 (MC solution), 10, 30 or 100 mg/kg (n = 8).

Rats reared under the same conditions as Test Example 1 were orally administered the compound, and 1 hour later, the rats were intraperitoneally injected with 0.5 ml of the triple anesthetic mixture per 100 g of rat body weight. A filter paper whose weight had been measured in advance was placed on the palm of each limb and tightly wrapped with plastic wrap or the like. Sweat secreted over a 30-minute period was absorbed and collected on the filter paper.

### (Results)

The weight of sweat collected over the 30-minute period is shown in Fig. 2. The compound had no effect on sweating even at 100 mg/kg.

### [Example 2: Evaluation of Sweating Effect of Compound in Combination with Pilocarpine]

The compound was suspended in MC solution immediately before use to prepare a dosing solution. The dosage was 0 (MC solution), 10, 30 or 100 mg/kg (n = 8). In addition, a dosing solution containing pilocarpine hydrochloride was prepared in the same manner as in Test Example 1 immediately before use.

Rats reared under the same conditions as Test Example 1 were orally administered the compound, and 1 hour later, the rats were intraperitoneally injected with 0.5 ml of the triple anesthetic mixture per 100 g of rat body weight. Sweat was collected over a 30-minute period in the same manner as in Example 1 (without pilocarpine), and then a dosing solution containing pilocarpine hydrochloride was subcutaneously administered (3 mg/kg). Five minutes later, sweat secreted over the 30-minute period was absorbed and collected on the filter paper (with pilocarpine). Statistical comparisons were performed using paired T-tests with and without pilocarpine.

### (Results)

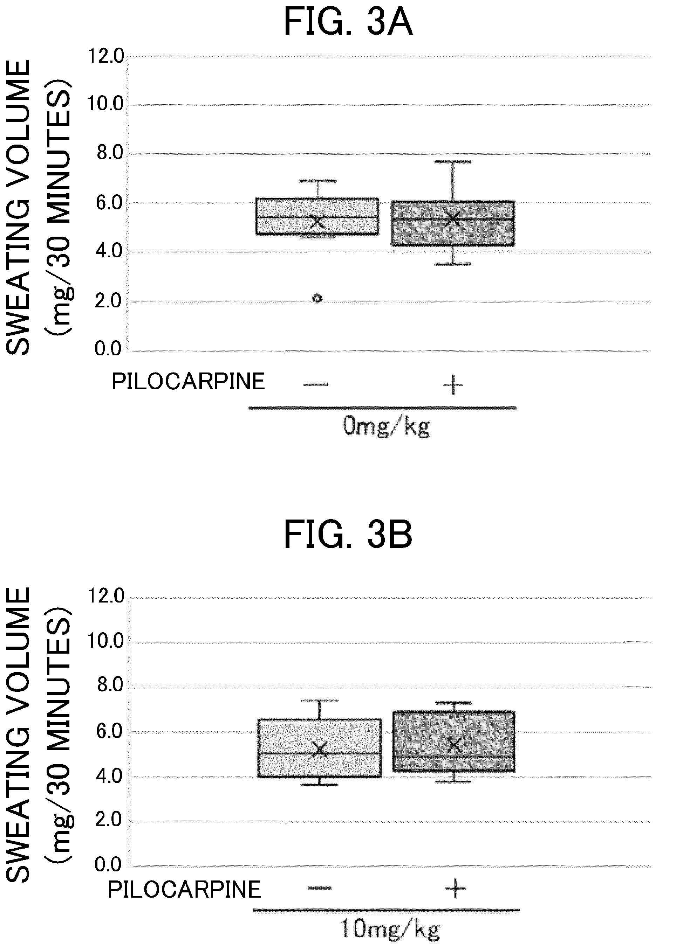

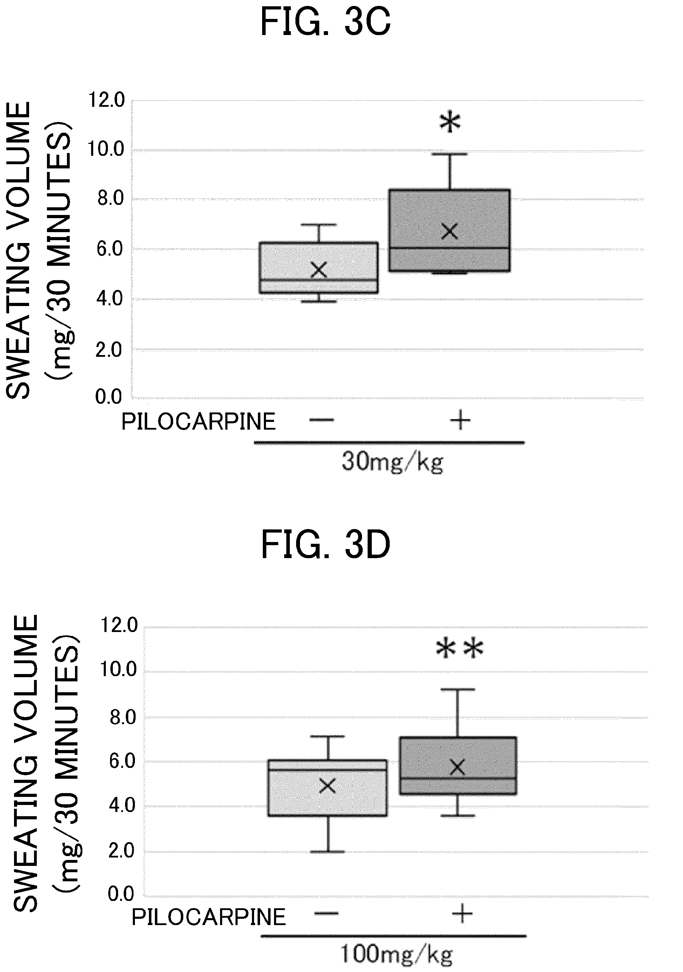

The weights of sweat collected from rats administered the compound at dosages of 0 (MC solution), 10, 30 and 100 mg/kg are shown in Figs. 3A, 3B, 3C and 3D, respectively. At 10 mg/kg, no significant change in the sweating effect induced by the compound was observed. On the other hand, at both 30 mg/kg and 100 mg/kg, a significant increase in sweating volume induced by the compound was observed (*p = 0.032, **p = 0.019). When administered in combination with pilocarpine hydrochloride, the compound increased sweating volume in a dosage-dependent manner.

### [Example 3: Evaluation of Renal Excretion Rate of Compound]

After a 21-day screening period, non-elderly male and female subjects were admitted to a clinical unit one day prior to compound administration. The subjects were randomized on the second day after the admittance and divided into 7 groups, each having 8 subjects. In each group, 6 subjects received the compound, and 2 subjects received a corresponding placebo. The dosage of the compound was set at 1, 3, 10, 30, 100, 150, or 200 mg. A single oral dose of the compound or placebo was administered to the subjects under fasting conditions, and then urine was collected continuously over the next 72 hours. Tween (trademark)-20 was added to the collected urine samples, and the urine samples containing 0.5% Tween (trademark)-20 were stored in the dark at -20°C until an unchanged compound measurement.

The unchanged compound was quantified by liquid chromatography-tandem mass spectrometry (LC-MS/MS). The calibration range of measurement is 1.00 ng/mL to 1000 ng/mL. 20 µL of an internal standard solution (500 ng/mL), 20 µL of orthophosphoric acid, 500 µL of water, and 100 µL of the urine sample were mixed and subjected to solid-phase extraction using an Oasis-MCX µElution 96-well plate (supplied by Waters Corporation). The mixture was then dissolved in 0.4 mL of reconstitution solution (50% acetonitrile solution of 25 mmol/L ammonium acetate), and 1 µL of the solution was injected into a column (Inertsil (trademark) ODS-3, 3 µm, 3.0 × 50 mm (supplied by GL Sciences Inc.)) and quantitatively analyzed using a Sciex 6500 mass spectrometer (supplied by SCIEX).

### (Results)

Table 1 shows the renal excretion rate of the unchanged compound in urine. The renal excretion rate of the compound was very low, ranging from 0.875% to 1.64%, indicating that the excretion rate from the kidney is low.

**[Table 1]**

| **Dosage(mg)** | | | | | | | |
|---|---|---|---|---|---|---|---|
| | **1 (n=6)** | **3 (n=6)** | **10 (n=6)** | **30 (n=6)** | **100 (n=5)** | **150 (n=5)** | **200 (n=5)** |
| **Renal Excretion Rate (%)** | | | | | | | |
| **Average (Standard Deviation)** | **0.875 (0.184)** | **0.981 (0.485)** | **1.02 (0.417)** | **1.45 (0.312)** | **1.25 (0.630)** | **1.53 (0.314)** | **1.64 (0.341)** |
| **%Coefficient of Variation** | **21.1** | **49.5** | **40.9** | **21.4** | **50.5** | **20.4** | **20.8** |
| **Median** | **0.927** | **0.916** | **0.960** | **1.49** | **1.03** | **1.50** | **1.65** |
| **Minimum - Maximum** | **0.650-1.06** | **0.488-1.66** | **0.571-1.70** | **1.06-1.87** | **0.781-2.49** | **1.16-2.03** | **1.12-2.05** |

The above-described embodiments are intended to illustrate the present invention and are not intended to limit the scope of the present invention. In other words, the scope of the present invention is indicated by the claims, not by the embodiments. Various modifications made within the scope of the claims and the equivalents thereof are considered to fall within the scope of the present invention.

The present application is based on Japanese Patent Application No. 2022-197009, filed on December 9, 2022. The entire description, claims, and drawings of Japanese Patent Application No. 2022-197009 are incorporated in the present description by reference.

### Industrial Applicability

The present invention is useful for pharmaceuticals, especially for body fluid regulators.

## Claims

1. A body fluid regulator comprising: a compound represented by Formula (I), an ester of the compound, or a salt of the compound,

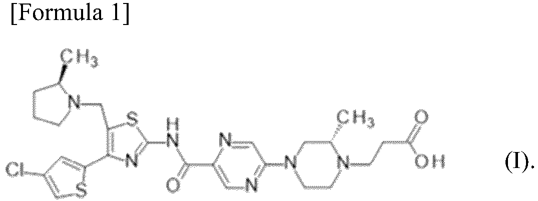

2. The body fluid regulator according to claim 1, administered in combination with a diuretic to a subject.

3. The body fluid regulator according to claim 1, administered in combination with an M₃ muscarinic receptor agonist to a subject.

4. The body fluid regulator according to claim 1, administered in combination with a thermal stimulation or a thermogenesis stimulation applied to a subject.

5. The body fluid regulator according to any one of claims 2 to 4, wherein
the subject suffers from a disease associated with excessively retained body fluid.

6. The body fluid regulator according to claim 5, wherein
the disease is heart failure, hypertension, cirrhosis, renal disease, nephrotic syndrome, renal failure, diabetes, drug-induced excess body fluid retention, lipedema, or lymphedema.

7. The body fluid regulator according to claim 5, wherein
the disease is renal failure, and the body fluid regulator is used to delay an initiation of dialysis therapy.

8. The body fluid regulator according to any one of claims 2 to 4, wherein
the subject suffers from a disease accompanied by skin dryness or pruritus.

9. The body fluid regulator according to claim 8, wherein
the disease is senile xerosis, atopic dermatitis, psoriasis, ichthyosis, radiation dermatitis, actinic keratosis, senile keratosis, renal disease, renal failure, or diabetes.

10. A body fluid regulating combination agent comprising:
a compound represented by Formula (I), an ester of the compound, or a salt of the compound; and
a diuretic,

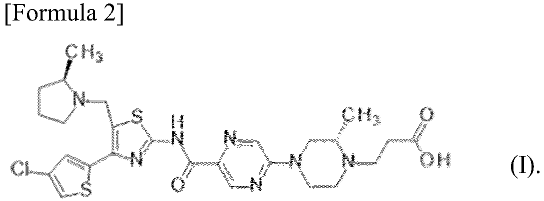

11. A body fluid regulating combination agent comprising:
a compound represented by Formula (I), an ester of the compound, or a salt of the compound; and
an M₃ muscarinic receptor agonist,

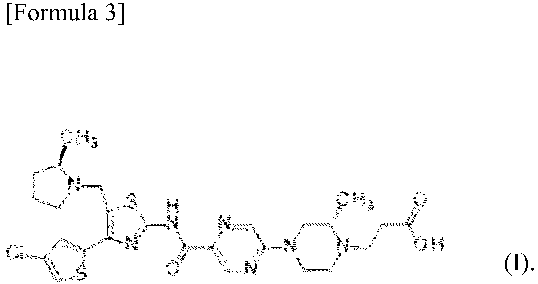

12. A use of a compound represented by Formula (I), an ester of the compound, or a salt of the compound for production of a body fluid regulator,
